Europäisches Patentamt

European Patent Office

Office européen des brevets

(19)

(11) Numéro de publication: **0 319 372 B1**

(12) **FASCICULE DE BREVET EUROPEEN**

(45) Date de publication de fascicule du brevet: **04.08.93**

(51) Int. Cl.⁵: **C12P 19/04**

(21) Numéro de dépôt: **88402930.7**

(22) Date de dépôt: **23.11.88**

(54) **Procédé de production de polysaccharides.**

(30) Priorité: **04.12.87 FR 8716855**

(43) Date de publication de la demande:
**07.06.89 Bulletin 89/23**

(45) Mention de la délivrance du brevet:
**04.08.93 Bulletin 93/31**

(84) Etats contractants désignés:
**AT BE CH DE ES FR GB GR IT LI LU NL SE**

(56) Documents cités:
**EP-A- 0 149 915
GB-A- 2 165 549
US-A- 3 427 226**

(73) Titulaire: **RHONE-POULENC CHIMIE**
**25, quai Paul Doumer**
**F-92408 Courbevoie Cédex(FR)**

(72) Inventeur: **Cros, Patrick**
**1, rue des Monnaies Collectif Champs Persé**
**F-79500 Melle(FR)**

(74) Mandataire: **Dubruc, Philippe et al**
**RHONE-POULENC INTERSERVICES Service**
**Brevets Chimie 25, quai Paul-Doumer**
**F-92408 Courbevoie Cédex (FR)**

Rank Xerox (UK) Business Services
(3. 10 / 3.6 / 3.3. 1)

**Description**

La présente invention a pour objet un procédé amélioré pour la production de polysaccharides par fermentation de carbohydrates au moyen de microorganismes. Plus spécifiquement, l'invention concerne un procédé de fermentation utilisant un amidon ou un hydrolysat d'amidon comme source de carbone nutritive.

Les polysaccharides de fermentation de poids moléculaire élevé ou biopolymères son utilisés de manière croissante dans de nombreuses applications industrielles pour leurs propriétés épaississantes, viscosifiantes, stabilisantes dans les milieux aqueux. Ainsi, la gomme Xanthane, en raison de ses propriétés rhéologiques exceptionnelles, est utilisée dans des domaines aussi variés que le bâtiment, la peinture, le papier, le textile, les cosmétiques, l'alimentation, l'agriculture, le traitement des eaux, le forage, la récupération du pétrole et autres.

Les biopolymères sont produits par culture aérobie de microorganismes dans un milieu nutritif aqueux.

La gomme xanthane est produite par des bactéries du genre Xanthomonas. Des biopolymères du même type peuvent être générés par une grande variété de microorganismes incluant, parmi les plus notoires, ceux du genre Agrobacterium, Arthrobacter, Alcaligenes (Succinoglycane), Pseudomonas (Levan), Rhizobium, Sclerotium (Scleroglucane). Ces polysaccharides ont des poids moléculaires élevés, le plus souvent supérieur à $1.10^6$ et sont constitués d'unités glucose, mannose, galactose, rhamnose, acide glucuronique, acide mannuronique, acide guluronique, avec éventuellement des dérivés acétate et pyruvate. Leur structure particulière et leurs propriétés sont décrites par exemple dans l'ouvrage Industrial Gums - Whistler - 2nd Edition - Chapters XXI-XXIII (1973).

Il existe de nombreuses publications concernant la production de polysaccharides de fermentation. Des procédés pour la production de gomme xanthane sont décrits par exemple dans les brevets US-A-3 020 206, 3 251 749, 3 391 060, 3 271 267, 3 427 226, 3 433 708, 3 455 786, 3 485 719, 3 594 280, 4 154 654, 4 282 321.

Le milieu nutritif aqueux contient normalement, outre des éléments de croissance variés, des hydrates de carbone assimilables comme source de carbone. Des hydrates de carbone convenables comprennent le glucose, le saccharose, le fructose, le maltose, le lactose, les amidons solubles et leurs hydrolysats. Bien que l'amidon brut soit décrit comme source de carbone convenable, il a pour inconvénient majeur d'allonger considérablement le cycle de fermentation par rapport aux monosaccharides tels que le glucose. En outre le microorganisme n'est pas capable de consommer la totalité des sucres réducteurs. La presence de ces sucres résiduels en fin de fermentation d'une part rend le milieu propice au développement de souches contaminantes pouvant dégrader le moût avant séparation du polysaccharide, et d'autre part risque de colorer le produit lors des traitements thermiques de pasteurisation et clarification éventuels.

La présente invention a pour but principal de proposer un procédé de fermentation économique, utilisant l'amidon comme source de carbone, et ayant une productivité au moins égale à celle obtenue avec le glucose ou les hydrolysats d'amidon à contenu élevé en glucose.

Il a été trouvé que les polysaccharides pouvaient être produits de manière économique en effectuant la fermentation au moyen du microorganisme producteur simultanément à l'hydrolyse enzymatique de l'amidon. Le procédé permet de manière inattendue d'obtenir un polysaccharide ayant des propriétés rhéologiques améliorées par rapport à celui obtenu sur amidon brut. D'autres avantages résultent de la diminution de la durée des fermentations, de la suppression des dextrines résiduelles de bas poids moléculaire et d'une productivité améliorée.

Le procédé de production de polysaccharides selon l'invention par fermentation aérobie de microorganismes dans un milieu nutritif aqueux comprenant de l'amidon comme source de carbone assimilable est caractérisé en ce que l'on conduit la fermentation additionnellement en présence d'au moins une enzyme amylolytique saccharifiante.

L'amidon utilisé comme source de carbone selon l'invention peut être n'importe quel amidon de céréales comme l'amidon de blé, de maïs, de sorgho, de riz, de tapioca, de seigle, d'avoine, ou un amidon de tubercules comme l'amidon de pomme de terre.

Le terme "amidon" inclut selon la présente description l'amidon brut en dispersion aqueuse et les hydrolysats d'amidon résultant de l'hydrolyse incomplète de l'amidon comme l'amidon fluidifié, les sirops d'amidon et les hydrolysats riches en dextrose. Les hydrolysats d'amidon diffèrent entre eux par leur degré d'hydrolyse, exprimé en équivalent dextrose D.E. et leur contenu en oligosaccharides et polysaccharides plus élevés. Les amidons fluidifiés présentent un D.E. compris entre environ 3 et 20 et renferment généralement 50 à 95 % de polysaccharides, de degré de polymérisation supérieur à G 7 (7 unités glucose). Les sirops d'amidon ou sirops de glucose de faible équivalent dextrose présentent un D.E. allant de environ 20 à 68 avec 10 à 50 % de polysaccharides supérieurs à G 7. Les hydrolysats d'amidon ou sirops riches en dextrose ont un D.E. pouvant atteindre 90-98 %. La préparation des hydrolysats d'amidon est bien connu dans la technique. Conventionnellement, les amidons fluidifiés et les sirops d'amidon sont obtenus par hydrolyse acide et/ou enzymatique ou moyen d'une $\alpha$ amylase de liquéfaction, éventuellement suivi d'une $\beta$ amylase. Pour les hydrolysats riches en glucose, l'amidon est le plus souvent transformé selon un procédé en 2 étapes, par action d'une $\alpha$ amylase de liquéfaction puis par action d'une enzyme de saccharification comme la glucoamylase, connue aussi sous le nom d'amyloglucosidase.

Dans la pratique du procédé selon l'invention on utilisera de préférence un sirop d'amidon et encore plus préférentiellement un amidon fluidifié. L'emploi des hydrolysats riches en glucose n'est pas intéressant du point de vue économique étant donné qu'il implique la saccharification préalable qui est une étape longue, même dans les conditions d'activité maximales de l'amyloglucosidase qui se situent à une température supérieure à 50°C et à pH de 4,5-5.

L'amidon et ses produits d'hydrolyse peuvent être employés directement dans le procédé de l'invention sous forme non purifiée, après stérilisation. On peut bien entendu mettre en oeuvre des produits commerciaux purifiés, concentrés ou déshydratés comme les maltodextrines.

L'amidon est présent dans le milieu de fermentation en quantité nécessaire pour fournir 1 à 15 % en poids de glucose par rapport au milieu de fermentation. Exprimé en amidon brut, sur une base sèche, des quantités convenables peuvent être comprises entre 5 et 200 g/l, de préférence entre 10 et 150 g/l du milieu de fermentation.

Les enzymes amylolytiques de saccharification que l'on ajoute selon le procédé de l'invention dans le milieu de fermentation contenant le microorganisme producteur d'un polysaccharide de haut poids moléculaire sont capables de transformer les dextrines de l'amidon en glucose et maltose. Comme enzymes de saccharification on peut citer les $\alpha$ amylases saccharifiantes comme l'$\alpha$ amylase de Bacillus subtilis var.amylosaccharitiens, l'$\alpha$ amylase fongique, les $\beta$ amylases, la glucoamylase, l'isoamylase, la pullulanase. Ces enzymes peuvent être employées seules ou en mélange entre elles.

On donne la préférence à la glucoamylase du fait de sa haute spécificité. La glucoamylase peut être toute glucoamylase fongique telle que celles appartenant aux genres Aspergillus, Endomyces ou Rhizopus. Dans le cas notamment où l'on utilise l'amidon brut comme source de carbone, il est encore possible d'employer un enzyme liquéfiante en addition à l'enzyme saccharifiante, par exemple un mélange $\alpha$ amylase liquéfiante - $\beta$ amylase ou $\alpha$ amylase liquéfiante - glucoamylase. Des préparations enzymatiques industrielles sont décrites dans l'ouvrage Encycl. of Pol. Sc. Vol 6, pp 46-53.

L'enzyme amylolytique de saccharification, éventuellement de liquéfaction, est ajoutée dans le milieu de fermentation en quantité nécessaire pour effectuer la saccharification, respectivement la fluidification, de l'amidon. La quantité minimale utile est fonction de l'activité de l'enzyme, de la quantité et du D.E. de l'amidon présent dans le milieu et peut être déterminée facilement par l'homme de métier. De manière générale, on utilise des quantités suffisantes pour fournir 0,02 à 4 unités d'activité enzymatique, de préférence 0,1 à 2 unités, par gramme d'amidon (exprimé en matières sèches). A titre d'exemple d'enzyme saccharifiante, l'AMG 200 L®, commercialisée par NOVO INDUSTRY et qui est une glucoamylase, peut être ajoutée en quantité de 0,01 à 2 %, de préférence entre 0,05 et 1 % en poids basé sur le poids de solides contenu dans l'amidon liquéfié présent dans le milieu de fermentation.

Le procédé de l'invention est susceptible d'être appliqué à la production de tout polysaccharide exocellulaire par fermentation de glucides au moyen de microorganismes. De nombreux microorganismes tels que bactéries, levures, champignons, algues, sont capables de produire des polysaccharides exocellulaires. On peut citer entre autres :

-   des bactéries appartenant au genre Xanthomonas et plus particulièrement aux espèces décrites dans Bergey's manual of determinative bacteriology (8e édition - 1974 - Williams N. Wilkins C° Baltimore) telles que Xanthomonas begoniae, Xanthomonas Campestris, Xanthomonas carotae, Xanthomonas hederae, Xanthomonas incanae, Xanthomonas malvacearum, Xanthomonas papavericola, Xanthomonas phaseoli, Xanthomonas pisi, Xanthomonas vasculorum, Xanthomonas vesicatoria, Xanthomonas vitians, Xanthomonas pelargonii, les bactéries appartenant au genre Arthrobacter et plus particulièrement les espèces Arthrobacter stabilis, Arthrobacter viscosus ; au genre Erwinia ; au genre Azotobacter et plus particulièrement l'espèce Azotobacter indicus; au genre Agrobacterium et plus particulièrement les espèces Agrobacterium radiobacter, Agrobacterium rhozigènes, Agrobacterium tumefaciens ; au genre Alcaligenes et plus particulièrement Alcaligenes faecalis ; au genre Pseudomonas et plus particulièrement Pseudomonas methanica ; au genre Corynebacterium ; au genre Bacillus et plus particulièrement Bacillus polymyxa ;
-   des champignons appartenant au genre Sclerotium et plus particulièrement aux espèces Sclerotium glucanicum, Sclerotium rolfsii ou Plectania occidentalis.
-   des levures appartenant au genre Hansenula comme l'espèce Hansenula capsulata.

En dehors de la source de carbone et de l'enzyme amylolytique utilisés selon l'invention, le milieu de fermentation et les conditions de la fermentation peuvent être choisis parmi ceux décrits dans la littérature pour chaque microorganisme. Des milieux de fermentation appropriés sont décrits par exemple dans l'ouvrage Chemicals by fermentation - Sydney J. Gutcho - Noyes Data Corp - 1973.

Un milieu de fermentation aqueux typique comprend, en dehors de la source de carbone, une source d'azote organique et/ou minérale comme l'extrait soluble de maïs (CSL) et/ou de soja, l'extrait de levure, les peptones, la gélatine, la caséine, les sels d'ammonium, tels que le chlorure d'ammonium, le nitrate d'ammonium, les carbonates d'ammonium, les sulfates d'ammonium, les nitrates comme le nitrate de sodium ou le nitrate de potassium. Le milieu de fermentation peut contenir en outre une source de phosphore assimilable qui est introduite dès le départ ou lors de la régulation du pH au cours de la fermentation, par exemple sous forme d'ions $PO_4^{---}$, ainsi qu'une source de magnésium comme le sulfate de magnésium, l'acétate de magnésium, le chlorure de magnésium, le nitrate de magnésium, et des traces d'oligoéléments essentiels pour la croissance et la multiplication, dont la nature dépend de la souche des microorganismes utilisés.

En ce qui concerne les modalités pratiques pour la conduite du procédé, on peut se reporter à la littérature existante, et notamment aux procédés décrits dans les brevets cités pour la préparation de la gomme Xanthane. Dans la plupart des cas, les détails de la production des polysaccharides à l'aide des autres microorganismes énumérés ci-dessus sont très voisins de ceux de la gomme xanthane.

D'une manière générale, le microorganisme est introduit dans le milieu de fermentation de manière connue en soi et par exemple à l'aide de cultures intermédiaires obtenues dans des fermenteurs de laboratoire de 20 litres qui elles mêmes ont été obtenues à partir d'inoculums réalisés par exemple en erlenmeyer d'un litre.

La préparation des inoculums ou des cultures intermédiaires connue de tout homme de l'art est décrite par exemple dans le brevet FR-A-2 414 555.

La fermentation est ensuite réalisée pendant quelques jours dans le milieu aéré et agité en une ou plusieurs étapes de croissance et de production. Le milieu de croissance et le milieu de production peuvent avoir la même composition ou une composition différente. La température d'incubation et le temps requis pour générer un pourcentage acceptable de polysaccharide varient naturellement selon le microorganisme utilisé. La température se situe généralement aux environs de 30°C ± 10°C. Dans la plupart des cas, le pH est maintenu dans le domaine de 6,0 à 7,5 et de préférence de 6,5 à 7,2. On peut utiliser un système régulateur de pH introduisant dans le milieu, si nécessaire, un réactif alcalin tel que la soude, la potasse, l'ammoniaque. Dans certains cas cependant, le rendement peut être optimum dans une gamme de pH inférieure. Par exemple, pour la production de scléroglucane, le rendement est optimum dans la gamme de pH initial de 3,5 à 5,5 (FR-A-1 386 287).

Afin d'obtenir une fermentation rapide, il est essentiel que le milieu soit suffisamment aéré et agité pour fournir la quantité correcte d'oxygène disponible pour la culture bactérienne que l'on fait croître. Les besoins de la fermentation en oxygène sont adaptés de manière conventionnelle aux conditions de la fermentation et du transfert d'oxygène. A cet égard, on notera que le procédé de l'invention peut être adapté aux procédés de fermentation en milieu émulsionné, tels que décrits dans EP-A-58364, EP-A-98474, EP-A-187092.

Après achèvement de la fermentation, le moût contenant le polysaccharide est traité de manière connue en soi. On procède généralement à une pasteurisation pour tuer les cellules microbiennes. Si on le désire, le moût peut être amené à subir des traitements thermiques et/ou enzymatiques et/ou de filtration destinés à améliorer les propriétés rhéologiques, de clarification et de filtrabilité. Il peut encore être avantageux dans certains cas de procéder à une concentration du moût qui peut être réalisée par tout moyen conventionnel.

Le polysaccharide peut être isolé du moût selon toute méthode usuelle telle que la précipitation au moyen d'un solvant dans lequel le produit est insoluble, par exemple un alcool inférieur, de préférence l'isopropanol et/ou à l'aide d'un sel minéral convenable. Le polysaccharide précipité est ensuite filtré, lavé, séché et broyé.

Le procédé décrit peut bien évidemment être réalisé en discontinu ou en continu par introduction continue du milieu stérile dans le récipient de fermentation.

On comprendra que la description ci-dessus vise un procédé spécifique pour préparer des biopolymères par fermentation à l'aide de microorganismes d'un milieu contenant une source d'hydrate de carbone produit à partir d'amidon, en présence d'enzymes capables d'hydrolyser l'amidon ou ses produits de dégradation en mono- et disaccharides et que l'invention elle-même ne se limite pas aux composés précis du milieu de fermentation, ni aux modes de réalisation particuliers.

4

Les bouillons entiers de fermentation et les poudres de polysaccharides sont utilisables dans toutes les applications connues des hydrocolloïdes. Les solutions aqueuses obtenues par dilution du moût ou solubilisation de la poudre se sont révélées posséder des propriétés rhéologiques supérieures à celles obtenues par dilution, dans les mêmes concentrations, d'un polysaccharide produit au moyen d'amidon brut ou d'amidon liquéfié, selon les procédés de l'art antérieur.

Les exemples suivants illustrent l'invention :

Exemple 1

Préparation de l'inoculum :

A partir d'une culture de Xanthomonas campestris maintenue sur gélose en tube on ensemence, dans un flacon agité, 100 ml d'un milieu contenant extrait de levure, extrait de malt, bactopeptone et 10 g/l d'amidon de blé. Le milieu ensemencé après stérilisation est incubé 24 h à 28°C. Le contenu d'un flacon sert à inoculer 15 l du milieu de fermentation.

Préparation de l'amidon brut fluidifié

On prépare un lait d'amidon de blé contenant 30 % en poids de matières sèches. Le pH est ajusté à 7 et l'on ajoute une enzyme de fluidification (TERMAMYL 120 L® - Novo Industry), à raison de 0,15 % en poids par rapport à l'amidon exprimé en matières sèches. La température est portée à 85°C et maintenue à cette valeur pendant 30 min. L'amidon fluidifié est stérilisé 30 min. à 121°C.

Fermentation

Essai A

Dans un fermenteur de 20 l, on prépare 15 l d'un milieu de production stérile ayant la composition suivante :

| Amidon (préparé ci-dessus) | 45 g/l (en matières sèches) |
|---|---|
| Farine de soja | 5,1 g/l |
| $MgSO_4$, $7H_2O$ | 0,25 g/l |
| Eau distilée | q.s.p. 1 litre |
| pH ajusté à 7 | |

On ajoute une glucoamylase (AMG 200 L® de Novo Industry) à un taux de 1 % par rapport à l'amidon exprimé en matières sèches. Immédiatement, après l'ajout de l'enzyme, le milieu est inoculé à l'aide de l'inoculum prépare ci-dessus.

La température est régulée à 28°C et le pH maintenu à 6,8-7,0 par addition automatique de soude. L'air est injecté dans le fermenteur avec une aération initiale de 40 VVH portée à 55 VVH lorsque la viscosité du milieu commence à augmenter. Le milieu est agité par 3 étages de pales Rushton à une vitesse de 200 à 400 rpm.

La fermentation est arrêtée quand il ne reste plus de sucres. Le moût est pasteurisé, puis le polysaccharide est précipité par addition d'isopropanol, filtré et séché 30 min. à 120°C.

Essai B (comparatif)

On opère dans des conditions identiques à celles de l'essai A, sauf que l'on n'ajoute pas de glucoamylase dans le milieu de production. La fermentation est arrêtée quand la concentration en sucres résiduels n'évolue plus.

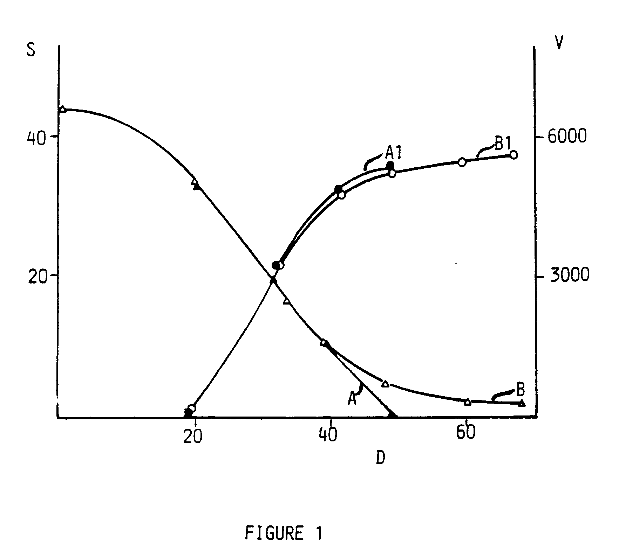

Au cours de chacun des essais A et B, des échantillons du moût sont prélevés à intervalles réguliers pour mesurer la quantité d'amidon résiduel dans le milieu. La mesure est faite par chromatographie liquide à haute performance après hydrolyse acide de l'amidon. On mesure également la viscosité du milieu (viscosimètre Brookfield, aiguille 4, 30 rpm, 20°C). Les résultats sont représentés graphiquement sur la Figure 1, où les courbes A, A1 et B, B1 représentent la quantité d'amidon résiduel (S) en g/l et la viscosité du milieu (V) en mPa.s en fonction de la durée (D) en heures de la fermentation des essais A et B respectivement. Les résultats de la fermentation sont donnés ci-après :

|  | Essai A | Essai B |
|---|---|---|
| . Durée de la fermentation | 50 h | > 66 h |
| . Sucres résiduels en fin de fermentation | 0 | > 2 g/l |
| . Matières sèches précipitables | 31,3 g/kg | 29,2 g/kg |
| . Rendement sur amidon brut | 69,6 % | 64,9 % |

Le pouvoir viscosifiant des produits est mesuré dans chaque cas sur des solutions aqueuses diluées préparées à partir de la poudre à une concentration de 0,2 % dans l'eau distillée (Brookfield - Aig. 1 20°C).

|  | Essai A | Essai B |
|---|---|---|
| 6 t/min | 600 mPa.s | 350 mPa.s |
| 30 t/min | 190 mPa.s | 135 mPa.s |

Exemples 2 et 3

On réalise 2 fermentations de la manière décrite à l'exemple 1 en utilisant les mêmes milieux et les mêmes conditions opératoires. Après constitution du milieu de production, on ajoute 0,25 % (exemple 2) ou 0,5 % (exemple 3) d'enzyme AMG 200 L®/amidon en matières sèches.

Les résultats sont reportés ci-dessous :

|  | Exemple 2 | Exemple 3 |
|---|---|---|
| Matières sèches précipitables | 30 g/kg | 29,7 g/kg |
| Durée de la fermentation | 66 h | 50 h |
| Rendement/amidon brut | 65,7 % | 66 % |
| Viscosité (mPa.s) | | |
| sol à 0,2 %   6 t/min | 375 | 575 |
| 30 t/min | 150 | 192 |

Exemple 4

On réalise une fermentation de la manière décrite à l'exemple 1 avec un inoculum identique et un amidon fluidifié préparé dans les mêmes conditions.

Le milieu de production a la composition suivante :

| Amidon fluidifié | 100 g/l |
|---|---|
| Farine de soja brute | 7,0 g/l |
| $MgSO_4,7H_2O$ | 0,25 g/l |
| Eau distillée | q.s.p. 1 litre |

Après constitution du milieu de production et avant inoculation, on ajoute 0,5 % de glucoamylase AMG 200 L® par rapport au poids d'amidon exprimé en matières sèches. Les conditions de la fermentation sont identiques à celles de l'exemple 1.

On obtient les résultats suivants, en comparaison avec un essai effectué dans des conditions identiques, mais en l'absence de glucoamylase.

|  | Exemple 4 | Comparatif |
|---|---|---|
| Matières sèches précipitables | 54 g/kg | 49,5 g/kg |
| Durée de la fermentation | 130 h | > 160 h |
| Sucres résiduels en fin de fermentation | 0 | > 15 g/l |
| Rendement/amidon | 54 | 49,5 |

L'essai comparatif étant non extractible étant donné le sucre résiduel élevé, le pouvoir viscosifiant n'a pas été mesuré.

## Revendications

1. Procédé de production de polysaccharides par fermentation aérobie de microorganismes dans un milieu nutritif aqueux comprenant de l'amidon comme source de carbone caractérisé en ce que l'on ajoute au milieu de fermentation contenant le microorganisme producteur dudit polysaccharide, au moins une enzyme amylolytique saccharifiante de manière à conduire la fermentation en présence de ladite enzyme.

2. Procédé selon la revendication 1 caractérisé en ce que l'amidon est utilisé en quantité nécessaire pour fournir 1 à 15% de glucose par rapport au milieu de fermentation.

3. Procédé selon les revendications 1 ou 2 caractérisé en ce que l'amidon est choisi dans le groupe des amidons fluidifiés et des sirops d'amidon.

4. Procédé selon les revendications 1 ou 2 caractérisé en ce que l'amidon est un amidon brut.

5. Procédé selon la revendication 4 caractérisé en ce que l'on ajoute une enzyme de liquéfaction en addition à l'enzyme saccharifiante.

6. Procédé selon l'une des revendications 1 à 5 caractérisé en ce que l'enzyme saccharifiante est choisie parmi les $\alpha$ amylases, les $\beta$ amylases, la glucoamylase, l'isoamylase, la pullulanase et leurs mélanges.

7. Procédé selon l'une des revendications 1 à 6 caractérisé en ce que l'enzyme saccharifiante est utilisée en quantité suffisante pour fournir 0,02 à 4 unités d'activité enzymatique par g d'amidon exprimé en matière sèche.

8. Procédé selon l'un des revendications 1 à 7 caractérisé en ce que l'enzyme saccharifiante est une glucoamylase et est utilisée en quantité comprise entre 0,01 et 2%, de préférence entre 0,05 et 1% en poids basé sur le poids de solides contenus dans l'amidon.

9. Procédé selon la revendication 7 caractérisé en ce que l'enzyme est utilisée en quantité suffisante pour fournir 0,1 à 2 unités d'activité enzymatique par g d'amidon mis en oeuvre.

10. Procédé selon l'une des revendications 1 à 9 caractérisé en ce que le microorganisme est choisi dans le groupe des bactéries du genre Xanthomonas, du genre Alcaligenes, du genre Agrobactérium, du genre Arthrobacter, du genre Azotobacter, du genre Pseudomonas, du genre Corynebactérium, des champignons du genre Sclérotium et des levures du genre Hansenula.

## Claims

1. Process for producing polysaccharides, by aerobic fermentation by microorganisms in an aqueous nutrient medium comprising starch as a carbon source, characterized in that at least one saccharifying amylolytic enzyme is added to the fermentation medium containing the microorganism producing the said polysaccharide so as to bring about fermentation in the presence of the said enzyme.

2. Process according to claim 1, characterized in that the starch is used in a quantity necessary for providing 1 to 15% of glucose relative to the fermentation medium.

**3.** Process according to claim 1 or 2, characterized in that the starch is chosen from the group of liquidized starches and starch syrups.

**4.** Process according to claim 1 or 2, characterized in that the starch is a crude starch.

**5.** Process according to claim 4, characterized in that a liquefying enzyme is added in addition to the saccharifying enzyme.

**6.** Process according to one of claims 1 to 5, characterized in that the saccharifying enzyme is chosen from $\alpha$-amylases, $\beta$-amylases, glucoamylase, isoamylase, pullulanase and mixtures thereof.

**7.** Process according to one of claims 1 to 6, characterized in that the saccharifying enzyme is used in a quantity sufficient for providing 0.02 to 4 units of enzyme activity per g of starch, expressed as dry matter.

**8.** Process according to one of claims 1 to 7, characterized in that the saccharifying enzyme is a glucoamylase and is used in a quantity of between 0.01 and 2%, and preferably between 0.05 and 1%, by weight, based on the weight of solids contained in the starch.

**9.** Process according to claim 7, characterized in that the enzyme is used in a quantity sufficient for providing 0.1 to 2 units of enzyme activity per g of starch employed.

**10.** Process according to one of claims 1 to 9, characterized in that the microorganism is chosen from the group of bacteria of the genus Xanthomonas, the genus Alcaligenes, the genus Agrobacterium, the genus Arthrobacter, the genus Azotobacter, the genus Pseudomonas and the genus Corynebacterium, of fungi of the genus Sclerotium and of yeasts of the genus Hansenula.

**Patentansprüche**

**1.** Verfahren zur Herstellung von Polysacchariden durch aerobe Fermentation mit Hilfe von Mikroorganismen in einem wäßrigen Nährsubstrat, das Starke als Kohlenstoffquelle enthält, dadurch gekennzeichnet, daß man dem Fermentationsmilieu, das die Mikroorganismen zur Herstellung des besagten Polysaccharids enthält, mindestens ein zuckerbildendes Stärkeenzym zur Durchführung der Fermentation zusetzt.

**2.** Verfahren nach Anspruch 1, dadurch gekennzeichnet, daß die Stärke in der erforderlichen Menge eingesetzt wird, um 1 - 15 % Glukose bezogen auf das Fermentationsmilieu zu erhalten.

**3.** Verfahren nach einem der Ansprüche 1 oder 2, dadurch gekennzeichnet, daß die Stärke aus der Gruppe der verflüssigten Stärken und des Stärkesirups ausgewählt wird.

**4.** Verfahren nach einem der Ansprüche 1 oder 2, dadurch gekennzeichnet, daß die Stärke eine Rohstärke ist.

**5.** Verfahren nach Anspruch 4, dadurch gekennzeichnet, daß man ein verflüssigendes Enzym zusätzlich zu dem zuckerbildenden Enzym zusetzt.

**6.** Verfahren nach einem der Ansprüche 1 bis 5, dadurch gekennzeichnet, daß das zuckerbildende Enzym aus den $\alpha$-Amylasen, den $\beta$-Amylasen, der Glukoamylase, der Isoamylase, der Pullulanase und ihrer Mischungen gewählt wird.

**7.** Verfahren nach einem der Ansprüche 1 bis 6, dadurch gekennzeichnet, daß das zuckerbildende Enzym in einer ausreichenden Menge eingesetzt wird, um 0,02 - 4 Enzymeinheiten pro Gramm Stärke bezogen auf das Trockengewicht zu erhalten.

**8.** Verfahren nach einem der Ansprüche 1 bis 7, dadurch gekennzeichnet, daß das zuckerbildende Enzym eine Glukoamylase ist und in einer Menge eingesetzt wird, die zwischen 0,01 und 2 %, vorzugsweise zwischen 0,05 und 1 Gew.-% bezogen auf den Feststoffgehalt der Stärke liegt.

**9.** Verfahren nach Anspruch 7, dadurch gekennzeichnet, daß das Enzym in einer ausreichenden Menge eingesetzt wird, um 0,1 - 2 Enzymeinheiten pro Gramm eingesetzter Stärke zu erreichen.

**10.** Verfahren nach einem der Ansprüche 1 bis 9, dadurch gekennzeichnet, daß die Mikroorganismen aus der Bakteriengruppe der Gattung Xanthomonas, der Gattung Alcaligenes, der Gattung Agrobacterium, der Gattung Arthrobacter, der Gattung Azotobacter, der Gattung Pseudomonas, der Gattung Corynebacterium, aus der Gruppe der Pilze der Gattung Sclerotium und aus der Gruppe der Hefen der Gattung Hansenula gewählt werden.

FIGURE 1